# EUROPEAN PATENT SPECIFICATION

(11) **EP 3 399 963 B1**
(45) Date of publication and mention of the grant of the patent: **07.06.2023**
(21) Application number: 17736445.2
(22) Date of filing: 06.01.2017
(51) Int. Cl.: A61K 9/06, A61K 9/00, A61K 47/10, A61K 47/38, A61K 47/14, A61K 31/568, A61K 31/56, A61K 31/196

(54) **GEL COMPOSITIONS FOR TRANSDERMAL DELIVERY TO MAXIMIZE DRUG CONCENTRATIONS IN THE STRATUM CORNEUM AND SERUM AND METHODS OF USE THEREOF**
GELZUSAMMENSETZUNGEN ZUR TRANSDERMALEN VERABREICHUNG ZUR MAXIMIERUNG DER ARZNEIMITTELKONZENTRATION IN DER STRATUM CORNEUM UND SERUM UND VERFAHREN ZUR VERWENDUNG DAVON
COMPOSITIONS DE GEL POUR ADMINISTRATION TRANSDERMIQUE PERMETTANT DE MAXIMISER LA CONCENTRATION DE MÉDICAMENTS AU NIVEAU DU STRATUM CORNEUM ET DU SÉRUM, ET MÉTHODES D'UTILISATION DE CES DERNIÈRES

(30) Priority: 07.01.2016 US 201662275955 P; 05.08.2016 US 201662371670 P
(43) Date of publication of application: 14.11.2018
(60) Divisional application: 22179555.2
(73) Proprietor: Viramal Limited, London W1S 1DN (GB)
(72) Inventor: BOLOGNA, William, New york, New York 10023 (US); LARSEN, Finn, Bronchester Bridge Hawick TD9 8JS (GB)
(74) Representative: Holme Patent A/S
(86) International application number: PCT/US2017/012564
(87) International publication number: WO 2017/120492

(56) References cited:
- WO-A1-99/62497
- WO-A1-2012/156820
- WO-A1-2014/026707
- US-A1- 2011 158 920
- US-A1- 2011 158 920
- US-A1- 2011 195 036
- US-A1- 2014 161 889
- US-A1- 2014 161 889
- US-A1- 2014 186 278
- US-B1- 6 187 323
- US-B1- 6 187 323
- ZHAOLU ZHU ET AL: "The development of polycarbophil as a bioadhesive material in pharmacy", ASIAN JOURNAL OF PHARMACEUTICAL SCIENCES, vol. 8, no. 4, 1 August 2013 (2013-08-01), pages 218-227, XP055609543, NL ISSN: 1818-0876, DOI: 10.1016/j.ajps.2013.09.003

## Description

This application claims priority to US provisional application number 62/275,955, filed on January 7, 2016; and US provisional application number 62/371,670 filed August 5, 2016.

Gel compositions comprising an active ingredient are disclosed in US 2011/158920 A1, US 2014/161889 A1, WO 99/62497 A1, US 6,18,323 B1, US 2011/195036 A1, and WO2012/156820 A1.

### SUMMARY

The invention is set out in the appended claims.

Disclosed herein are gel compositions comprising: (a) at least one active ingredient or a salt thereof; (b) at least one oleogel comprising at least one oily agent and at least one lipid soluble cellulose polymer, where the at least one oily agent can comprise from about 5 % to about 40 % by weight of the total weight of the composition and the at least one cellulose polymer can comprise from about 1 % to about 10 % by weight of the total weight of the composition; and (c) at least one aqueous gel; where the active agent is micronized; the composition can comprise a bioadhesive; the composition can comprise at most about 4 % w/w of an alcohol, where the alcohol can be ethanol or isopropanol; or any combination thereof.

In some embodiments, the composition can comprise a concentration of an alcohol of at most about 4 % w/w, and where the alcohol can be a C₁-C₈ alcohol. In some cases, the C₁-C₈ alcohol can be ethanol or isopropanol. In another embodiment, the concentration of alcohol can be about 3.5 % w/w.

The at least one active ingredient is dispersed or suspended in at least a portion of the at least one aqueous gel. In some embodiments, the lipid soluble cellulose polymer can be an alkyl cellulose. In some cases, the alkyl cellulose can be selected from the group consisting of methylcellulose, ethylcellulose, hydroxypropylcellulose, and combinations thereof. In another embodiment, the lipid soluble cellulose polymer can be an alkyl carboxylic containing cellulose or a salt thereof. In some cases, the alkyl carboxylic acid containing cellulose can be non-sodium containing carboxymethylcellulose. In some embodiments, the alkyl cellulose can comprise from about 1 % to about 10 % by weight of the total weight of the composition. In another embodiment, the ethylcellulose can comprise from about 1 % to about 10 % by weight of the total weight of the composition. In another embodiment, the alkyl carboxylic containing cellulose or a salt thereof can comprise from about 1 % to about 10 % by weight of the total weight of the composition. In another embodiment, the non-sodium containing carboxymethyl cellulose can comprise from about 1 % to about 10% by weight of the total weight of the composition.

In some embodiments, the oily agent can be selected from the group consisting of: a monoglyceride, a diglyceride, a triglyceride, and any combination thereof. In some cases, the oily agent can be isolated and purified. In some cases, the oily agent can be selected from the group consisting of: a synthetic diglyceride; a synthetic triglyceride; a propylene glycol isostearate; a polyoxy ethyl enated oleic glyceride mixture; an oil of plant origin; and any combination thereof.

In some embodiments, the propylene glycol isostearate can comprise from about 0.2 % to about 2 % by weight of the total weight of the composition. In some embodiments, the polyoxyethylenated oleic glyceride mixture can comprise from about 0.2 % to about 2 % by weight of the total weight of the composition.

The active ingredient is micronized. In some embodiments, the composition can comprise a bioadhesive. In some embodiments, the active ingredient is micronized and the composition can comprise a bioadhesive.

The active ingredient is selected from estradiol or a salt thereof, testosterone or a salt thereof, levonorgestrel or a salt thereof, ethinylestradiol or a salt thereof, and/or diclofenac or a salt thereof.

In some embodiments, the active ingredient can comprise from about 0.00001 % to about 10 % by weight of the total weight of the composition. In some embodiments, the active ingredient can be estradiol or a salt thereof. In some cases, the estradiol or salt thereof can comprise from about 0.025 % to about 5 % by weight of the total weight of the composition. In another embodiment, the active ingredient can comprise testosterone or a salt thereof. In some cases, the testosterone or salt thereof can comprise from about 0.1 % to about 6 % by weight of the total weight of the composition. In another embodiment, the active ingredient can comprise levonorgestrel or a salt thereof. In some cases, the levonorgestrel or salt thereof can comprise from about 0.00001 % to about 2 % by weight of the total weight of the composition. In another embodiment, the active ingredient can comprise ethinylestradiol or a salt thereof. In some cases, the ethinylestradiol or salt thereof can comprise from about 0.00001 % to about 2 % by weight of the total weight of the composition. In another embodiment, the active ingredient can comprise a combination of ethinylestradiol and levonorgestrel, salts thereof, or any combination thereof. In some cases, the ethinylestradiol or salt thereof can comprise from about 0.00001 % to about 2 % by weight of the total weight of the composition and the levonorgestrel or salt thereof can comprise from about 0.00001 % to about 2 % by weight of the total weight of the composition. In another embodiment, the active ingredient can comprise diclofenac or a salt thereof. In some cases, the diclofenac or salt thereof can comprise from about 0.1 % to about 6 % by weight of the total weight of the composition.

In some embodiments, the composition can comprise no more than about 4 % of a penetration enhancer by weight of the total weight of the composition. In some embodiments, the composition can comprise no more than about 2 % of a surfactant by weight of the total weight of the composition. In some cases, the surfactant can be selected from the group consisting of non-ionic, cationic, amphoteric, zwitterionic, and any combination thereof.

In some embodiments, the aqueous gel further can comprise at least one gelling agent. In some cases, the at least one gelling agent can be selected from the group consisting of a carbomer; a poloxamer; sodium carboxymethylcellulose; and a combination thereof. In some cases, the at least one gelling agent can comprise from about 0.1 % to about 10 % by weight of the total weight of the aqueous gel.

In some embodiments, the oleogel can be at a ratio of between about 10:90 to about 90: 1 by weight with respect to the aqueous gel. In some embodiments, the oleogel can comprise from about 10 % to about 30 % by weight of the total weight of the composition. In some embodiments, the aqueous gel can comprise from about 70 % to about 90 % by weight of the total weight of the composition.

In some embodiments comprising the bioadhesive, when the gel composition can be applied to a at least a portion of a skin of a subject, the gel composition can at least partially minimizes clumping of insoluble material relative to an otherwise identical composition lacking the at least one oleogel and the at least one aqueous gel. In some cases, the skin can be a vaginal skin.

In some embodiments, the bioadhesive can be selected from the group consisting of: a carbomer; glyceryl monooleate; hypromellose; polycarbophil; poly(methylvinyl ether-co-maleic anhydride); a salt thereof; and a combination thereof. In some cases, the bioadhesive can be polycarbophil, a salt thereof, or a combination thereof.

In some embodiments, the composition can maintain a stable uniform appearance over a period of about 1 year when stored in a sealed container, at about 25 °C, at about 1 atm pressure, and at about 50 % relative humidity.

In some embodiments, the composition can be a pharmaceutical composition. In some embodiments, the composition can be in unit dose form.

Also disclosed herein are gel compositions for use in at least partially ameliorating a condition comprising applying to at least a portion of a skin of a subject a gel composition described herein.

In some embodiments, the application of the composition to the portion of the skin of the subject can provide a reservoir of the active ingredient in the stratum corneum of the subject. In some cases, the reservoir can provide about a zero order release rate profile of a therapeutically effective amount of the active ingredient into a serum of the subject.

In some embodiments, the condition can be a hormone imbalance. In some cases, the hormone imbalance can comprise low testosterone. In some cases, the hormone imbalance can comprise low progesterone. In some cases, the hormone imbalance can comprise low estrogen. In another embodiment, the condition can be menopause. In another embodiment, the condition can be endometriosis. In another embodiment, the condition can be a cancer. In some cases, the cancer can be prostate cancer. In some cases, the cancer can be breast cancer. In some cases, the cancer can be cervical cancer. In another embodiment, the condition can be dry skin. In another embodiment, the condition can be arthritis. In some cases, the arthritis can be Rheumatoid arthritis. In another embodiment, the condition can be a migraine.

In some embodiments, the subject may have been previously diagnosed with or can be suspected of having a cancer.

In some embodiments, an addition pharmaceutical substance can be co-administered. In some cases, the additional pharmaceutical substance can be an antineoplastic. In some cases, the additional pharmaceutical substance can be an anti-inflammatory.

In some embodiments, the skin can be a vaginal skin. In some embodiments, the subject can be a human. In some embodiments, the subject can be in need thereof.

Also disclosed herein are methods of providing a reservoir of at least one active ingredient or a salt thereof in a stratum corneum of a subject comprising applying to at least a portion of a skin of the subject a gel composition comprising: (a) at least one active ingredient or a salt thereof; (b) at least one oleogel comprising at least one oily agent and at least one lipid soluble cellulose polymer; and (c) at least one aqueous gel; where the application of the composition to the at least a portion of the skin of the subject can provide a reservoir of the active ingredient in the stratum corneum of the subject.

In some embodiments, the reservoir can provide about a zero order release rate profile of the at least one active ingredient into a serum of the subject.

Also disclosed herein are methods of providing a reservoir of at least one active ingredient or a salt thereof in a stratum corneum of a subject comprising applying to at least a portion of a skin of the subject a gel composition described herein; where the application of the composition to the at least a portion of the skin of the subject can provide a reservoir of the active ingredient in the stratum corneum.

Also disclosed herein are methods of increasing absorption of a topically applied active ingredient into a serum of a subject comprising applying to at least a portion of a skin of the subject a gel composition described herein; where the application of the gel composition to the skin of the subject can increase absorption into the serum of the subject relative to an otherwise identical composition comprising penetration enhancers in an amount sufficient to increase penetration of the active ingredient in the skin of the subject.

Also disclosed herein are methods of reducing clumping of insoluble bioadhesive material in a vaginal delivery formulation comprising administering a gel composition described herein to a subj ect in need thereof.

Also disclosed herein are methods of preparing a gel composition comprising: (a) dispersing at least one micronized active ingredient in an aqueous gel; and (b) contacting the dispersion of (a) with at least one oleogel gel to form an a gel composition; where the at least one oleogel can comprise at least one oily agent and at least one lipid soluble cellulose polymer.

In some embodiments, the at least one oily agent can comprise from about 5 % to about 40 % by weight of the total weight of the composition and the at least one cellulose polymer can comprise from about 1 % to about 10 % by weight of the total weight of the composition. In some embodiments, the method can further comprise dissolving an alcohol in at least a portion of the at least one aqueous gel, at least one oleogel, or a combination thereof; where the amount of alcohol can be at most about 4% w/w. In some cases, the alcohol can be ethanol or isopropanol. In some embodiments, the method can further comprise dispersing a bioadhesive into at least a portion of the aqueous gel, oleogel, or a combination thereof.

Also disclosed herein are kits comprising a container comprising a gel composition disclosed herein; where application of a unit dose of the gel composition to a skin of a subject can provide a therapeutically effective amount of an active ingredient into a serum of the subject. In some embodiments, the kit can further comprise instructions for use. In some cases, the instructions for use can instruct a subject to apply a unit dose of the gel composition to at least a portion of the skin of the subject.

Also disclosed herein are methods of making a kit comprising placing a gel composition described herein in a container. In some embodiments, the method can further comprise combining the container with instructions for use.

### BRIEF DESCRIPTION OF THE DRAWINGS

The invention is set out in the appended claims. A better understanding of features and advantages will be obtained by reference to the following detailed description that sets forth illustrative embodiments, in which exemplary principles are utilized, and the accompanying drawings of which:
Figs. 1 A and IB are graphs that measure testosterone absorption in subjects treated with an exemplary composition.
Figs. 2 A and 2B are graphs that measure testosterone absorption in subjects treated with a comparative prior art composition.
Fig. 3 is a graph that illustrates the results of testosterone levels measured from comparative tests of an exemplary composition and one that is representative of the prior art.
Fig. 4 is a graph that illustrates the results of estradiol levels measured from comparative tests of an exemplary composition and one that is representative of the prior art.
Fig. 5 is a graph that illustrates the results of testosterone levels measured from comparative tests of an exemplary composition and one that is representative of the prior art.
Figure 6A, 6B and 6C are microscopic images of three groups administered a control formulation (6A), a formulation representative of the prior art (6B) or an exemplary composition (6C).

### DETAILED DESCRIPTION

### Overview

A composition can be formulated to ensure that an active ingredient (drug) can be built up in the stratum corneum of a subject, whereby the stratum corneum acts an "reservoir" from which the active ingredient can be slowly released and enter the systemic circulation in a controlled manner. The inventors have discovered the surprising and unexpected result that this not only provides a method of sustained release of active ingredient without needing a complex drug delivery system, but also that an increased bioavailability of the active ingredient can also be obtained.

Disclosed herein are gel compositions comprising: (a) at least one active ingredient or a salt thereof; (b) at least one oleogel comprising at least one oily agent and at least one lipid soluble cellulose polymer, where the at least one oily agent can comprise from about 5% to about 40% by weight of the total weight of the composition and the at least one cellulose polymer can comprise from about 1 % to about 10 % by weight of the total weight of the composition; and (c) at least one aqueous gel; where the active agent is micronized; the composition can comprise a bioadhesive; the composition can comprise at most about 4 % w/w of an alcohol, where the alcohol can be a C₁-C₈ alcohol; or any combination thereof.

Also disclosed herein are gel compositions for use in at least partially ameliorating a condition comprising applying to at least a portion of a skin of a subject a gel composition described herein.

Also disclosed herein are methods of providing a reservoir of at least one active ingredient or a salt thereof in a stratum corneum of a subject comprising applying to at least a portion of a skin of the subject a gel composition comprising: (a) at least one active ingredient or a salt thereof; (b) at least one oleogel comprising at least one oily agent and at least one lipid soluble cellulose polymer; and (c) at least one aqueous gel; where the application of the composition to the at least a portion of the skin of the subject can provide a reservoir of the active ingredient in the stratum corneum of the subject.

Also disclosed herein are methods of increasing absorption of a topically applied active ingredient into a serum of a subject comprising applying to at least a portion of a skin of the subject a gel composition described herein; where the application of the gel composition to the skin of the subject can increase absorption into the serum of the subject relative to an otherwise identical composition comprising penetration enhancers in an amount sufficient to increase penetration of the active ingredient in the skin of the subject.

Also disclosed herein are methods of reducing clumping of insoluble bioadhesive material in a vaginal delivery formulation comprising administering a gel composition described herein to a subj ect in need thereof.

Also disclosed herein are methods of preparing a gel composition comprising: (a) dispersing at least one micronized active ingredient in an aqueous gel; (b) contacting the dispersion of (a) with at least one oleogel gel to form an a gel composition; where the at least one oleogel can comprise at least one oily agent and at least one lipid soluble cellulose polymer.

Also disclosed herein are kits comprising a container comprising a gel composition described herein; where application of a unit dose of the gel composition to a skin of a subject can provide a therapeutically effective amount of an active ingredient into a serum of the subject.

### Definitions

The terminology used herein is for the purpose of describing particular cases only and is not intended to be limiting. As used herein, the singular forms "a", "an" and "the" can be intended to include the plural forms as well, unless the context clearly indicates otherwise. Furthermore, to the extent that the terms "including", "includes", "having", "has", "with", or variants thereof can be used in either the detailed description and/or the claims, such terms can be intended to be inclusive in a manner similar to the term "comprising".

The term "about" or "approximately" can mean within an acceptable error range for the particular value as determined by one of ordinary skill in the art, which will depend in part on how the value is measured or determined, i.e., the limitations of the measurement system. For example, "about" can mean about plus or minus 10%, per the practice in the art. Alternatively, "about" can mean a range of up to 20%, up to 10%, up to 5%, or up to 1% of a given value.

Alternatively, particularly with respect to biological systems or processes, the term can mean within an order of magnitude, within 5-fold, or within 2-fold, of a value. Where particular values may be described in the application and claims, unless otherwise stated the term "about" meaning within an acceptable error range for the particular value should be assumed. Also, where ranges and/or subranges of values are provided, the ranges and/or subranges can include the endpoints of the ranges and/or subranges.

The term "subject", "patient" or "individual" as used herein in reference to an individual, and can encompass a mammal and a non-mammal. A mammal can be any member of the Mammalian class, including but not limited to a human, a non-human primates such as a chimpanzee, an ape or other monkey species; a farm animal such as cattle, a horse, a sheep, a goat, a swine; a domestic animal such as a rabbit, a dog, and a cat; a laboratory animal including a rodent, such as a rat, a mouse and a guinea pig, and the like. A non-mammal can include a bird, a fish and the like. In some embodiments, a subject can be a mammal. In some embodiments, a subject can be a human.

The terms "treat," "treating", "treatment," "ameliorate" or "ameliorating" and other grammatical equivalents as used herein, can include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition, and can include prophylaxis. The terms can further include achieving a therapeutic benefit and/or a prophylactic benefit. Therapeutic benefit can mean eradication or amelioration of the underlying disease being treated. Also, a therapeutic benefit can be achieved with the eradication or amelioration of one or more of the physiological symptoms associated with the underlying disease such that an improvement can be observed in the patient, notwithstanding that, in some embodiments, the patient can still be afflicted with the underlying disease. For prophylactic benefit, a composition can be administered to a patient at risk of developing a particular disease, or to a patient reporting a physiological symptom of a disease, even if a diagnosis of the disease has not been made.

The terms "administer," "administering", "administration," and the like, as used herein, can refer to methods that can be used to enable delivery of compounds or compositions to the desired site of biological action. These methods can include oral administration, intraduodenal administration, parenteral administration (including intravenous, subcutaneous, intrathecal, intraperitoneal, intramuscular, intravascular or infusion), topical and rectal administration. In some instances, a subject can administer the gel composition in the absence of supervision. In some instances, a subject can administer the gel composition under the supervision of a medical professional (e.g., a physician, nurse, physician's assistant, orderly, hospice worker, etc.).

The terms "effective amount", "therapeutically effective amount" or "pharmaceutically effective amount" as used herein, can refer to a sufficient amount of a compound being administered which will at least partially ameliorate a symptom of a disease or condition being treated.

The terms "pharmaceutically acceptable salt" or simply "salt" as used herein, can refer to a salt that retains at least some of the biological effectiveness of the free acids and bases of the specified compound. In some instances, the salt can be not biologically or otherwise undesirable. In some embodiments, a compound disclosed herein can possess acidic or basic groups and therefore can react with any of a number of inorganic or organic bases, and inorganic and organic acids, to form a pharmaceutically acceptable salt. In some embodiments, a salt can be prepared in situ during the final isolation and purification of a compound, or by separately reacting a purified compound in its free base form with a suitable organic or inorganic acid, and isolating the salt thus formed.

Examples of pharmaceutically acceptable salts can include those salts prepared by reaction of a compound disclosed herein with a mineral, organic acid or inorganic base, such salts can include, acetate, acrylate, adipate, alginate, aspartate, benzoate, benzenesulfonate, bisulfate, bisulfite, bitartrate, bromide, butyrate, butyn-1,4-dioate, camphorate, camphorsulfonate, caproate, caprylate, chlorobenzoate, chloride, citrate, cyclopentanepropionate, decanoate, digluconate, dihydrogenphosphate, dinitrobenzoate, dodecyl sulfate, ethanesulfonate, formate, fumarate, glucoheptanoate, glycerophosphate, glycolate, hemisulfate, heptanoate, hexanoate, hexyne-1,6-dioate, hydroxybenzoate, γ-hydroxybutyrate, hydrochloride, hydrobromide, hydroiodide, 2-hydroxyethanesulfonate, iodide, isobutyrate, lactate, maleate, malonate, methanesulfonate, mandelate, metaphosphate, methanesulfonate, methoxybenzoate, methylbenzoate, monohydrogenphosphate, 1-napthalenesulfonate, 2-napthalenesulfonate, nicotinate, nitrate, palmoate, pectinate, persulfate, 3 -phenyl propionate, phosphate, picrate, pivalate, propionate, pyrosulfate, pyrophosphate, propiolate, phthalate, phenyl acetate, phenylbutyrate, propanesulfonate, salicylate, succinate, sulfate, sulfite, succinate, suberate, sebacate, sulfonate, tartrate, thiocyanate, tosylate, undeconate and xylenesulfonate.

The term "pharmaceutical composition," or simply "composition" as used herein, can refer to a biologically active compound, optionally mixed with at least one pharmaceutically acceptable chemical component, such as a carrier, a stabilizer, a diluent, a dispersing agent, a suspending agent, a thickening agent, an excipient and the like.

The term "carrier" as used herein, can refer to a relatively nontoxic chemical compound or agent that facilitates the incorporation of a compound into cells or tissues.

The terms "pharmaceutical combination", "administering an additional therapy", "administering an additional therapeutic agent" "administering a second compound" and the like, as used herein, can refer to a pharmaceutical therapy resulting from the mixing or combining of more than one active ingredient and can include both fixed and non-fixed combinations of a compound or composition disclosed herein. The term "fixed combination" can mean that at least one of a compound disclosed herein, and at least one co-agent, can both be administered to a patient simultaneously in the form of a single entity or dosage. The term "non-fixed combination" can mean that at least one of a compound disclosed herein, and at least one co- agent, can be administered to a patient as separate entities either simultaneously, concurrently or sequentially with variable intervening time limits, wherein such administration can provide effective levels of the two or more compounds in the body of the patient. These can also apply to cocktail therapies, e.g. the administration of three or more active ingredients.

The terms "co-administration", "administered in combination with" and their grammatical equivalents or the like, as used herein, can encompass administration of selected therapeutic agents to a single patient, and can include treatment regimens in which the agents can be administered by the same or different route of administration or at the same or different times. In some embodiments, a compound disclosed herein can be co-administered with other agents. These terms can encompass administration of two or more agents to an animal so that both agents and/or their metabolites can be present in the animal at the same time. They can include simultaneous administration in separate compositions, administration at different times in separate compositions, and/or administration in a composition in which both agents can be present. Thus, in some embodiments, a compound and another agent(s) can be administered in a single composition. In some embodiments, a compound and another agent(s) can be admixed in the composition.

The term "bioavailability" can refer to a fraction of an administered dose of unchanged active ingredient, e.g. a drug, that reaches the systemic circulation.

The term "zero order release rate profile" or "zero order release profile" can refer to a profile in which a concentration of an active ingredient can be released into a serum of a subject at a constant rate over a given time interval.

The term "substantially free," can be used to indicate certain ingredients that do not need to be included in a composition or mixture. The amount of ingredient can be so small that it does not cause irritation or generate an odor that could be objectionable to a subject. In some instances, the amount by weight of these ingredients can be less than about 5%, less than about 4.5%, less than about 4%, less than about 3.5%, less than about 3%, less than about 2.5%, less than about 2%, less than about 1.5%, less than about 1%, or less than about 0.5%. In some cases, the amount by weight of these ingredients can be less than about 0.9%, less than about 0.8%, less than about 0.7%, less than about 0.6%, less than about 0.5%, less than about 0.4%, less than about 0.3%), less than about 0.2%, or less than about 0.1%. In some cases, the amount by weight of these ingredients can be less than about 0.09%, less than about 0.08%, less than about 0.07%, less than about 0.06%, less than about 0.05%, less than about 0.04%, less than about 0.03%, less than about 0.02%, or less than about 0.01%. In some instances, the amount by weight of these ingredients can be from about 0.1% to about 5%, from about 0.1% to about 4.5%, from about 0.1%) to about 4%), from about 0.1% to about 3.5%, from about 0.1% to about 3%, from about 0.1%) to about 2.5%), from about 0.1% to about 2%, from about 0.1% to about 1.5%, from about 0.1% to about 1%), or from about 0.1% to about 0.5%. In some cases, the amount by weight of these ingredients can be 0%.

The term "micronized" as used herein can refer to a form of an active ingredient in which the average particle size can be minimized to about the micrometer scale. In some instances, the average particle size can be minimized to about the nanometer scale. In some cases, the average particle size can be from about 0.001 nm to about 500 µm, from about 0.001 nm to about 400 µm, from about 0.001 nm to about 300 µm, from about 0.001 nm to about 200 µm, from about 0.001 nm to about 100 µm, from about 0.001 nm to about 90 µm, from about 0.001 nm to about 80 µm, from about 0.001 nm to about 70 µm, from about 0.001 nm to about 60 µm, from about 0.001 nm to about 50 µm, from about 0.001 nm to about 40 µm, from about 0.001 nm to about 30 µm, from about 0.001 nm to about 20 µm, from about 0.001 nm to about 10 µm, from about 0.001 nm to about 5 µm, from about 0.001 nm to about 1 µm, from about 0.001 nm to about 900 nm, from about 0.001 nm to about 800 nm, from about 0.001 nm to about 700 nm, from about 0.001 nm to about 600 nm, from about 0.001 nm to about 500 nm, from about 0.001 nm to about 400 nm, from about 0.001 nm to about 300 nm, from about 0.001 nm to about 200 nm, from about 0.001 nm to about 100 nm, from about 0.001 nm to about 90 nm, from about 0.001 nm to about 80 nm, from about 0.001 nm to about 70 nm, from about 0.001 nm to about 60 nm, from about 0.001 nm to about 50 nm, from about 0.001 nm to about 40 nm, from about 0.001 nm to about 30 nm, from about 0.001 nm to about 20 nm, from about 0.001 nm to about 10 nm, from about 0.001 nm to about 5 nm, from about 0.001 nm to about 1 nm, from about 0.001 nm to about 0.9 nm, from about 0.001 nm to about 0.8 nm, from about 0.001 nm to about 0.7 nm, from about 0.001 nm to about 0.6 nm, from about 0.001 nm to about 0.5 nm, from about 0.001 nm to about 0.4 nm, from about 0.001 nm to about 0.3 nm, from about 0.001 nm to about 0.2 nm, from about 0.001 nm to about 0.1 nm, from about 0.001 nm to about 0.09 nm, from about 0.001 nm to about 0.08 nm, from about 0.001 nm to about 0.07 nm, from about 0.001 nm to about 0.06 nm, from about 0.001 nm to about 0.05 nm, from about 0.001 nm to about 0.04 nm, from about 0.001 nm to about 0.03 nm, from about 0.001 nm to about 0.02 nm, or from about 0.001 nm to about 0.01 nm. In some instances, the average particle size can be about 0.01 nm, about 0.05 nm, about 0.1 nm, about 0.15 nm, about 0.2 nm, about 0.25 nm, about 0.3 nm, about 0.35 nm, about 0.4 nm, about 0.45 nm, about 0.5 nm, about 0.55 nm, about 0.6 nm, about 0.65 nm, about 0.7 nm, about 0.75 nm, about 0.8 nm, about 0.85 nm, about 0.9 nm, about 0.95 nm, about 1 nm, about 2 nm, about 3 nm, about 4 nm, about 5 nm, about 6 nm, about 7 nm, about 8 nm, about 9 nm, about 10 nm, about 15 nm, about 20 nm, about 25 nm, about 30 nm, about 35 nm, about 40 nm, about 45 nm, about 50 nm, about 55 nm, about 60 nm, about 65 nm, about 70 nm, about 75 nm, about 80 nm, about 85 nm, about 90 nm, about 95 nm, about 100 nm, about 150 nm, about 200 nm, about 250 nm, about 300 nm, about 350 nm, about 400 nm, about 450 nm, about 500 nm, about 550 nm, about 600 nm, about 650 nm, about 700 nm, about 750 nm, about 800 nm, about 850 nm, about 900 nm, about 950 nm, about 1 µm, about µm, about 3 µm, about 4 µm, about 5 µm, about 6 µm, about 7 µm, about 8 µm, about 9 µm, about 10 µm, about 15 µm, about 20 µm, about 25 µm, about 30 µm, about 35 µm, about 40 µm, about 45 µm, about 50 µm, about 55 µm, about 60 µm, about 65 µm, about 70 µm, about 75 µm, about 80 µm, about 85 µm, about 90 µm, about 95 µm, about 100 µm, about 150 µm, about 200 µm, about 250 µm, about 300 µm, about350 µm, about 400 µm, about 450 µm, or about 500 µm.

The term "bioadhesive" as used herein can refer to a polymeric material that can create an intimate contact between an active ingredient and a biological substrate. The term "mucoadhesion" can be used interchangeably to describe bioadhesion to a mucus membrane.

The term "w/w" as used herein can refer to a weight of a component of a composition relative to the total weight of the composition.

### Formulation

It is generally accepted within the art, that if a drug (active ingredient) can be detained in the stratum corneum, absorption into the circulatory system can occur at an unacceptably low rate. Accordingly, research and conventional drug formulations have until now aimed at providing means for ensuring that the drug passes the stratum corneum as fast and effective as possible, e.g. by adding penetration enhancers to the respective formulations. In contrast, the inventors have discovered the surprising result that a composition with minimal penetration enhancers can ensure that the active ingredients can be not only detained but also accumulated in the stratum corneum, such that it can be the stratum corneum rather than the delivery system itself (as is the case with patches and the like) acts as the reservoir of drug for the entire dosing interval. Thereby, the need for a user to topically administer a composition several times a day and/or use expensive delivery systems, such as patches, can be eliminated. Thus, these gel compositions not only ensure that a maximization of drug can be delivered to the stratum corneum reservoir thereby creating a superior delivery system, but they can also provide increased patient compliance.

In some instances, the gel composition can be free from penetration enhancers or similar compounds that can facilitate a fast passage of the stratum corneum after the gel composition has been applied to a skin; thereby building up an active ingredient in the stratum corneum. Accordingly, the stratum corneum can function as a reservoir for an active ingredient.

In some instances, the gel composition can be free from surfactants. Surfactants can include non-ionic surfactants, cationic surfactants, amphoteric surfactants and zwitterionic surfactants. In some cases, the gel composition can be stable during prolonged storage without the use of such surfactants.

In some instances, the gel composition can comprise a bioadhesive. Examples of bioadhesives can include carbomers; glyceryl monooleate; hypromellose; polycarbophil; poly(methylvinyl ether-co-maleic anhydride); as well as salts thereof.

In some aspects, the bioadhesive can be polycarbophil or a salt thereof. Polycarbophil was designed to mimic negatively charged mucin, the glycoprotein component of mucus that is responsible for its attachment to underlying epithelial surfaces. Polycarbophil is a lightly cross- linked polymer. Polycarbophil is also a weak polyacid containing multiple carboxyl radicals (COO-) the source of its negative charges. These acid radicals can permit hydrogen bonding with a cell surface. Hydrogen bonds can be weak, in the case of polycarbophil they can be numerous. Bioadhesives such as polycarbophil can stay attached to vaginal epithelial cells until they turn over, which can be up to 7 days in menopausal women. However, vaginal clumping and discharge can occur due to the water insoluble polycarbophil remaining attached to the vaginal epithelial cells. The inventors discovered the surprising and unexpected result that a gel composition described herein comprising an oleogel and an aqueous gel can prevent or minimize vaginal clumping or discharge when formulated with a bioadhesive by emulsifying the insoluble material, thereby minimizing or eliminating unsightly clumping or discharge.,

In some instances, an aqueous gel of a gel composition described herein can comprise polycarbophil at a concentration by weight of at least about 0.01%, at least about 0.05%, at least about 0.1%, at least about 0.15%, at least about 0.2%, at least about 0.25%, at least about 0.3%, at least about 0.35%, at least about 0.4%, at least about 0.45%, at least about 0.5%, at least about 0.55%, at least about 0.6%, at least about 0.65%, at least about 0.7%, at least about 0.75%, at least about 0.8%, at least about 0.85%, at least about 0.9%, at least about 0.95%, at least about 1%), at least about 1.1%, at least about 1.2%, at least about 1.3%, at least about 1.4%, at least about 1.5%, at least about 1.6%, at least about 1.7%, at least about 1.8%, at least about 1.9%, at least about 2%, at least about 2.5%, at least about 3%, at least about 3.5%, at least about 4%, at least about 4.5%, at least about 5%, at least about 5.5%, at least about 6%, at least about 6.5%, at least about 7%, at least about 7.5%, at least about 8%, at least about 8.5%, at least about 9%, at least about 9.5%, or at least about 10%. In some instances, an aqueous gel of a gel composition described herein can comprise a polycarbophil at a concentration by weight of from about 0.1% to about 10%, from about 0.1% to about 9%, from about 0.1% to about 8%, from about 0.1% to about 7%, from about 0.1% to about 6%, from about 0.1% to about 5%, from about 0.1% to about 4%, from about 0.1% to about 3%, from about 0.1% to about 2%, from about 0.1% to about 1%, from about 0.1% to about 0.9%, from about 0.1% to about 0.8%, from about 0.1% to about 0.7%, from about 0.1% to about 0.6%, from about 0.1% to about 0.5%, from about 0.1% to about 0.4%, from about 0.1% to about 0.3%, or from about 0.1% to about 0.2%.

In some instances, a gel composition described herein can comprise an oleogel, an aqueous gel and a bioadhesive. A gel composition comprising an oleogel, an aqueous gel and a bioadhesive can be formulated to reduce or eliminate vaginal clumping or discharge by emulsifying the bioadhesive cell debris, thereby reducing or eliminating clumping or discharge.

In some instances, a gel composition described herein comprising an oleogel, an aqueous gel and a bioadhesive can be formulated as a vaginal delivery formulation. In some instances, the formulation can comprise an active ingredient or water to be delivered vaginally. This formulation can deliver the active ingredient or water to the subject while minimizing unsightly clumping or discharge.

The at least one active ingredient is dispersed or suspended in at least one aqueous gel.

If the active ingredient can be easily dissolved in alcohol, the gel composition may comprise a low concentration of alcohol in order to ensure that active ingredient(s) can be solubilized. In some embodiments, the alcohol can be a C₁-C₈ alcohol. Examples of a C₁-C₈ alcohol can include methanol, ethanol, n-propanol, isopropanol, t-butanol, pentanol, hexanol, cyclohexanol, heptanol, octanol and the like. In some cases, the gel composition can have an alcohol concentration of at most 5% by weight. In some cases, the alcohol concentration can be at most 4%) by weight. Alcohol in these concentrations will not function as a penetration enhancer, as is the case with alcohol in higher concentrations e.g. greater than 50 or 60 to 95% by weight, as e.g. in commercial hydroalcoholic testosterone and estradiol formulations where 60 to 70% alcohol is typically used as the principal penetration enhancer. In some cases, the alcohol concentration can be about 3.5%.

An oleogel can comprise:
(i) a cellulose polymer selected from an ethylcellulose, a non-sodium containing carboxymethylcellulose or a mixture thereof, and
(ii) an oily agent.

An oily agent can be selected from the group consisting of: a monoglyceride, a diglyceride, a triglyceride, and any combination thereof. In some cases, an oily agent can be isolated and purified. In some cases, an oily agent can be selected from the group consisting of: a synthetic diglyceride; a synthetic triglyceride; a propylene glycol isostearate; a polyoxyethylenated oleic glyceride mixture; an oil of plant or natural origin; and any combination thereof.

In some instances, a mono- di- or triglyceride can be a molecule of Formula I: 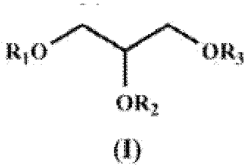 wherein R₁, R₂ , and R₃ can independently be H; or C₁-C₂₀ alkyl comprising 0, 1, 2, 3, 4 or 5 degrees of unsaturation.

In some aspects, the synthetic mono- di- or triglyceride can be "LABRAFAC^{®} lipophile WL1349", sold by the company Gatefosse, propylene glycol isostearate, such as the product sold under the name "hydrophilol isostearique" by the company Gatefosse, and the polyglycolyzed glyceride "LABRRAFIL^{®} M 1944 CS" as sold by Gatefosse.

LABRRAFIL^{®} M 1944 CS is a mixture of polyoxyethylenated oleic glycerides obtained by the alcoholysis of natural plant oil. It is an oily liquid whose properties are presented in table 1 below.

**Table 1**

| Chemical name | Polyglycolyzed oleic glyceride |
|---|---|
| Trade name | LABRRAFIL^{®} M 1944 CS |
| Drop Point °C | Liq. |
| Saponification Number | 1451175 |
| Acid number | > 2 |
| Iodine Number | 60/90 |
| Oral acute toxicity rat | OLD > 20mg/kg |
| LOP | 0 |
| HLB | 314 |

In some cases, a mono-, di- or triglyceride can be of natural or plant origin. An oil of natural or plant origin can include an oil such as sweet almond oil, argan oil or palm oil.

In some instances, the cellulose polymer can be a lipid soluble cellulose polymer. In some instance, the cellulose polymer can be an alkyl cellulose. In some instance, the alkyl cellulose can be methyl cellulose, ethylcellulose, hydroxypropylcellulose or a combination thereof. In some instances, the cellulose polymer can be an alkyl carboxylic containing cellulose or a salt thereof. In some cases, the alkyl carboxylic containing cellulose can be non-sodium containing carboxymethylcellulose.

In some instances, the cellulose polymer can be present in a proportion of 1 and about 10% by weight. In some instances, the cellulose polymer can be present in a proportion of 1 and about 10% by weight and the oily agent can comprise LABRRAFIL^{®} M 1944 CS. In some cases, the oily agent can be present in a proportion of between about 5 and 90% by weight, relative to the total weight of the oleogel. In some cases, the ratio of the oleogel to the weight of the aqueous gel can be from about 10:90 to about 90:10. In some cases, the cellulose polymer can be EMULFREE^{®} P. In some instances, the cellulose polymer can be EMULFREE^{®} P and the oily agent can comprise LABRRAFIL^{®} M 1944 CS.

The oily agent can further comprises propylene glycol isostearate, in a proportion of between of about 5 and 90% by weight, relative to the total weight of the oleogel.

The aqueous gel present in the gel composition can comprise at least one gelling agent selected from the group consisting of carbomers, poloxamers, sodium carboxymethylcellulose and mixtures of these. In some cases, the gelling agent can be present in a proportion of from about 0.1 to about 10% of the weight, relative to the weight of the aqueous gel. In some cases, the ratio of the weight of the oleogel to the weight of the aqueous gel can be from about 10:90 to about 90:10.

In some aspects, the gelling agent for the aqueous gel can be a carbomer, Carbopol 974 or Carbopol 980, present in a proportion of between of about 0.1 and about 5% by weight, relative to the total weight of the aqueous gel.

The oleogel and the aqueous gel can respectively further comprise standard ingredients for a gel, such as texture agents, antioxidants, preservatives, dyes or fragrances of various types and in conventional amounts which are known to not cause skin irritation. [0093] In some cases, a gel composition described herein can be a stable gel composition. In some instances, the gel composition can maintain uniform appearance over a period of about 1 year when stored in a sealed container, at about 25 °C, at about 1 atm pressure, and at about 50% relative humidity. In some instances, the gel composition can maintain uniform appearance for at least about 1.5 years, at least about 2 years, at least about 2.5 years, at least about 3 years, at least about 3.5 years, at least about 4 years, at least about 4.5 years, at least about 5 years, at least about 6 years, at least about 7 years, at least about 8 years, at least about 9 years, or about 10 years when stored in a sealed container, at about 25 °C, at about 1 atm pressure, and at about 50% relative humidity.

An active ingredient is in a micronizedform in the gel composition, and can in principal be any kind of locally or systematically active medicament/drug.

The active ingredients testosterone; estradiol; ethinylestradiol; levonorgestrel; diclofenac; a salt of any of these, and any combination thereof.

Irrespective of the active ingredient or the intended use, the gel composition can be adapted to accumulate the active ingredient in the stratum corneum and deliver pharmaceutically effective amounts of active ingredient(s) with a substantially zero order release profile, for a prolonged period of time. In some cases, the period of time can be at least about 4 hours, at least about 8 hours, at least about 12 hours, at least about 16 hours, at least about 20 hours, at least about 24 hours, at least about 36 hours, at least about 48 hours, or at least about 72 hours.

The term "pharmaceutically effective," can mean an amount which can be sufficient to effect the desired physiological or pharmacological change in the subject. This amount can vary depending upon such factors as the potency of the particular ingredient, the desired physiological or pharmacological effect, and the time span of the intended treatment. Those skilled in the arts will be able to determine the pharmaceutically effective amount for any given active ingredient(s) in accordance with standard procedures.

The gel composition can be in the form of a pharmaceutical composition. In some instances, the pharmaceutical composition can be administered similar to a cosmetic product in the form of a cream or gel that can be applied to the skin. In some instances, the composition can be in a unit dose form when applied to at least a portion of a skin in a specified amount. Since the pharmaceutical composition can comprise a stable mixture of an oleo-gel and an aqueous gel, it can be be neither messy nor watery, and in comparison to conventional hydroalcoholic gels, it requires a smaller area for application, and can be quicker to dry.

The reservoir effect and increased bioavailability in the mixture may not be dependent on traditional penetration enhancers. In fact, as demonstrated in the examples below, competitive high alcohol products can be superior in penetration but inferior in bioavailability. Unlike the long held belief in the art, superior penetration may not be a prerequisite for greater bioavailability.

Gel compositions lacking penetration enhancers can differ from traditional hydroalcoholic gels in the following ways in that they can be:
Safer - containing less than 3.5% alcohol content vs. 60-70%; i.e., gel composition can cause less skin irritation
Non-flammable (can be allowed on-board aircraft)
User friendly - with much less product required (about 40% less than lead competitors) for administration along with a much quicker drying time (no need to stand and dry)
Accepted better by patients or users because the mixtures may not be watery, messy or smelly
Better at delivering the active agent with improved bio-availability
Lower in content of active ingredient, which results in a reduced risk of environmental exposure

### Indications

Also disclosed herein are gel compositions for use in at least partially ameliorating a condition comprising applying to at least a portion of a skin of a subject a gel composition disclosed herein. In some instances, the condition can comprise a hormone imbalance. Examples of hormone imbalance can include low testosterone, low progesterone and low estrogen. In some instances, a gel formulation described herein can be used to provide a supplement of the deficient hormone in an amount sufficient to at least partially ameliorate the imbalance.

In some aspects, a partial amelioration can include a diminishing of symptoms of the hormone imbalance. Examples of symptoms that can present with low estrogen levels can include hot flashes, headaches, lowered libido, breast atrophy, reduced bone density leading to secondary osteoporosis and atrophic changes such as pH change in the vagina. Examples of symptoms that can present with low testosterone can include mood changes (low mood and irritability), poor concentration, low energy, reduced muscle strength, increased body fat, longer time to recover from exercise, lowered libido, difficulty getting and keeping erections, low semen volume, reduced beard or body hair growth, breast development (gynecomastia), hot flushes, sweats, and osteoporosis. Examples of symptoms that can present with low progesterone can include hot flushes, mood swings, insomnia, night sweats, vaginal dryness and restlessness.

In some instances, the condition can be menopause. Menopause can be defined as the absence of menstrual periods for 12 months. It can be a time in a woman's life when the function of the ovaries ceases. A partial amelioration of menopause can include a diminishing of symptoms of menopause. Symptoms of menopause can include abnormal vaginal bleeding, hot flashes, vaginal and urinary symptoms, vaginal dryness and mood changes. Complications that women may develop after menopause include osteoporosis and heart disease. In some cases, administration of a hormone such as estrogen or progesterone using a gel composition described herein can be used to at least partially ameliorate some of the symptoms of menopause. In some cases, administration of a gel composition comprising a bioadhesive as described herein can be advantageous in delivering an active ingredient or water to alleviate some of the symptoms of menopause.

In some instances, the condition can be endometriosis. Endometriosis can be an often painful disorder in which tissue that normally lines the inside of a uterus - the endometrium - grows outside the uterus (endometrial implant). Endometriosis most commonly involves the ovaries, bowel or the tissue lining a pelvis. Rarely, endometrial tissue may spread beyond a pelvic region. In endometriosis, displaced thickens, breaks down and bleeds with each menstrual cycle. Because this displaced tissue has no way to exit the body, it becomes trapped. Surrounding tissue can become irritated, eventually developing scar tissue and adhesions - abnormal tissue that binds organs together. In some instances, delivery of active ingredients using a gel formulation described herein can at least partially ameliorate the symptoms of endometriosis. In some aspects, the active ingredient can be a hormone or a steroid.

In some instances, the condition can be a cancer. In some instances, the cancer can be prostate cancer. In some forms of prostate cancer, treatment with a hormone such as estrogen can be used as an androgen suppression therapy to help diminish the effect of androgen hormones on the cancer cells. In some cases, administration of a gel composition described herein comprising a hormone such as estrogen can be used to help treat prostate cancer in a patient suffering therefrom. In some aspects, a hormone therapy can be used in combination with an antineoplastic drug to treat prostate cancer.

In some instances, an anti -inflammatory can be topically delivered to a patient suffering from arthritis to include Rheumatoid arthritis. Topical, localized delivery coupled with ease of use and enhanced bioavailability afforded by the gel composition described herein can be used to reduce the inflammation associated with arthritis in a subject suffering therefrom.

### Kits

Kits can comprise a gel composition described herein; wherein application of a unit dose of the gel composition to a skin of a subject provides a therapeutically effective amount of an active ingredient into a serum of the subject. In some aspects, the gel composition can be packaged in a container. In some aspects, the kit can further comprise instructions that direct a subject to apply a unit dose of the gel composition to a skin.

Methods of making the kit can include placing the gel composition described herein in a container for packaging. The method can further comprise the inclusion of instructions for use. In some cases, the instructions for use can direct a subject to apply a unit dose of the gel composition to a skin.

### Examples

A number of examples were conducted in order to compare the gel compositions with conventional preparations containing identical amounts of active ingredients.

**Example 1: Comparing permeability and bioavailability of testosterone in a conventional hydroalcoholic gel with an exemplary gel composition.** An exemplary gel composition, called TESTOCREAM herein, with the following composition was prepared:

### TESTOCREAM Mixture (%w/w):

Testosterone 1.0%
Carbomer (CARBOPOL ^{®} 980 NF) 1.4%
Sorbic Acid 0.2%
Ethanol 96.5° 3.5%
Sodium Methyl Paraben 0.25%
EMULFREE^{®} P 4.0%
Purified water USP/EP 69.57%
LABRAFAC^{®} lipophille WL 1349 20.0%
Sodium Hydroxide 0.08%
Total 100%

The TESTOCREAM composition was manufactured by dissolving testosterone in the oil phase consisting of LABRAFAC^{®} lipophille WL 1349 and EMULFREE^{®}, to this suspension alcohol was added. The carbomers were hydrated in the aqueous phase. After hydration glycerin, Sorbic acid and Sodium Methyl paraben were added to the aqueous phase. The two phases were mixed in an appropriate vessel. The provided TESTOCREAM mixture was neither messy nor watery, and could be applied to the skin of a subject in a similar manner as a cosmetic cream, i.e., the cream was quickly absorbed and did not leave any residues on the skin.

### In-vitro permeability

The permeation, absorption and serum concentrations of the TESTOCREAM composition was compared with a conventional hydroalcoholic gel, called TESTOGEL^{®} obtainable by Laboratories Besins International. The TESTOGEL^{®} product also contained 1% testosterone.

The test formulations were as follows:
A) TESTOCREAM Mixture. 4 aluminum foil sachets, coded "VML0403, 1638340, 15/JUN/2016"
B) TESTOGEL^{®} Product. 1 aluminum foil sachet coded "TESTOGEL^{®} 50 mg. 80798 L, 01.2016" hydroalcoholic gel

The permeation of the two formulations was tested in a standard in-vitro test using a standard flow-through Diffusion Cell technology from PermeGear Inc., Riegelsville, PA, USA.

Approximately 20 h before the start of exposure to the test preparation, split-thickness skin membranes was placed in 9 mm flow-through automated diffusion cells (PermeGear Inc., Riegelsville, PA, USA) to hydrate the skin. The target skin surface temperature was 32 + 1 °C and humidity was ambient.

The dose preparations was applied with a positive displacement pipette and subsequently spread evenly over the skin surface within the donor compartment using a disposable glass rod (dose volume *ca.* 10 mg.cm⁻² ).

Following application of the test preparations, the actual temperature was recorded at 15-minute intervals during the study in a diffusion cell containing a non-exposed skin membrane. The receptor fluid (phosphate buffered saline (PBS) supplemented with 0.01% (w/v) sodium azide and 5% (w/v) bovine serum albumin (BSA), pH 7.2) was pumped at a flow rate of *ca.* 1.8 mL.h⁻¹.

Exposure duration of the different compositions to the skin was 24 hours. The experimental design was as follows:

**Table 2**

| Test group | Group size | Formulation | Nominal concetration | Target dose (µg.cm⁻²) |
|---|---|---|---|---|
| A | 6 | VML0403 | 1 % (w/v) | 100 |
| B | 6 | Testogel^{®} | 1 % (w/v) | 100 |

Samples were collected at the following intervals: [0-1 h], [1-2 h], [2-4 h], [4-8 h], [8-12 h], [12-16 h], [16-20 h] and [20-24 h].

Test Concentrations and homogeneity check, and application: The calculations of the relative absorption into the receptor fluid, a nominal amount of 1 %, i.e. 10 mg.g⁻¹ was used.

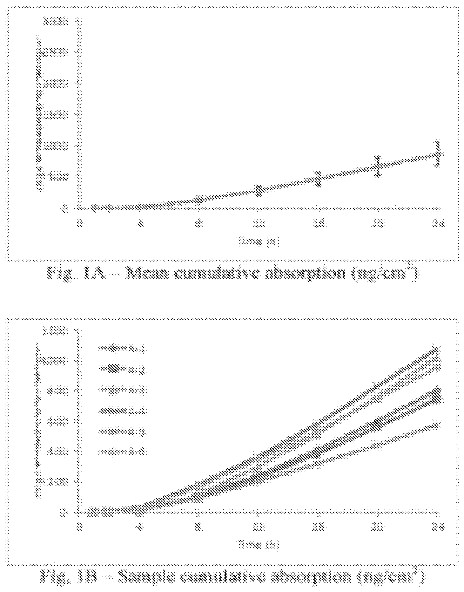

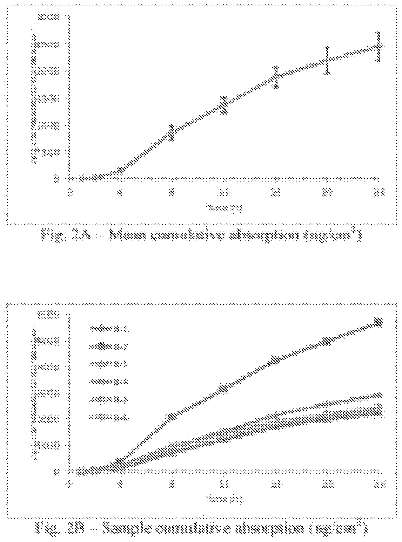

The results for the TESTOCREAM mixture, i.e. composition A) are shown in Figs. 1A and 1B and the results for the conventional TESTOGEL^{®} gel i.e. composition B) are shown in Figs. 2A and 2B. It is clear from the results that the TESTOCREAM composition had a lower level of permeation of testosterone than the hydroalcoholic gels when tested in-vitro in an established model.

### In-vivo bioavailability

The bioavailability of the TESTOCREAM composition, was then compared with the TESTOGEL^{®} product in a rat model.

A method including the conditions of administration of the drug on a defined surface area, the treatment of the animals and the assay of testosterone was developed.

The study found that the gel could be applied accurately on the skin of the animals using lmL prefilled syringes that the commercial assay method used for human works well, and that transdermal passage of testosterone in rats could be visualized. The method was then applied to two groups of rats which received transdermally the same quantity of either of the TESTOGEL^{®} or TESTOCREAM compositions.

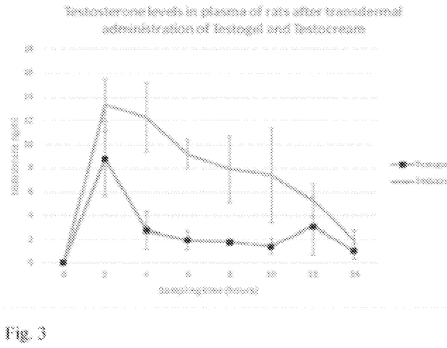

Blood was collected up to 24 h (8 time points) and testosterone levels were determined. The results are shown in Fig. 3.

As discussed earlier in relation to Figs. 1 and 2, the TESTOCREAM mixture had a lower level of permeation of testosterone than the TESTOGEL^{®} hydroalcoholic gel when tested in-vitro, which can be an accurate predictor of percutaneous absorption of drugs. Surprisingly and unexpectedly, however, the TESTOCREAM composition produced significantly higher absorption and serum concentrations of testosterone as is evident from Fig. 3, than the traditional hydroalcoholic gel when tested in-vivo.

Accordingly, this demonstrates that the TESTOCREAM mixture is surprisingly significantly more bioavailable than the TESTOGEL^{®} product despite having a lower rate of permeation through the skin. In particular, the TESTOCREAM mixture appears to be approximately 2.8 fold more bioavailable than the TESTOGEL^{®} product in the *in-vivo* rat model

Like all traditional hydroalcoholic transdermal gels, the TESTOGEL^{®} product relies on the principle that a solution will enhance drug permeation, by driving force, which enables molecules to across the stratum corneum without having to supersaturated increasing the better permeate use chemical permeation enhancers. However, once the alcohol has evaporated drug permeation ceases and the remainder of the dose remains on the surface of the skin as a dry residue.

By contrast a gel composition disclosed herein can maximize the amount of drug delivered and stored in the stratum corneum reservoir from the gel composition that are provided, e.g. the form of biphasic creams, gels, emulsions etc., and creates a superior delivery system. The vehicle does not evaporate and drug is not left on the surface of the skin. It is more efficiently delivered to the stratum corneum reservoir from which there can be controlled delivery of the drug to the vascular dermis and absorption into the systemic circulation.

The rat data has been confirmed in humans, similar serum concentrations of testosterone are achieved with a significantly lower dose (40 %) of the TESTOCREAM product than with hydroalcoholic gels.

The use of a gel composition described herein comprising testosterone can accordingly ensure that testosterone can build up in the stratum corneum thereby providing a testosterone reservoir. Testosterone has a half-life of 1.4 h ± 0.22 minutes [Cooper ER, Kasting GB. Transport across epithelial membranes. J Control Rel 1987; 6: 23-3] and the study showed that by administering a single daily dose it can be possible to maintain physiologic serum concentrations for a full 24 hours in hypogonadal men.

### Example 2: Comparing bioavailability of Estradiol in a conventional hydroalcoholic gel with an exemplary gel composition.

An exemplary gel composition, called ESTROCREAM herein, with the following composition was prepared in a manner similar to the TESTOCREAM composition.

### ESTROCREAM composition (%w/w):

Estradiol 0.1%
Ethanol 96.5° 5.0%
LABRAFAC0 lipophile WL1349 19.40%
EMULFREE0 P 4.0%
Purified water (1) 62.95%
CARBOPOL0 980 F 1.4%
Purified water (2) 3.3%
Sodium methylparaben 0.25%
Purified water (3) 3.315%
Sodium hydroxide 0.085%
Sorbic acid 0.2%

An *in-vivo* study similar to the one performed in Example 1 was conducted comparing the ESTROCREAM composition with a marketed hydroalcoholic gel called ESTROGEL^{®} which is obtainable from Besins Healthcare.

Using the same method as described in Example 1, the different compositions were applied to two groups of rats which received transdermally the same quantity of estradiol in either of the ESTROGEL^{®} or ESTROCREAM compositions. The blood was collected up to 24 h (8 time points) and estradiol levels were determined.

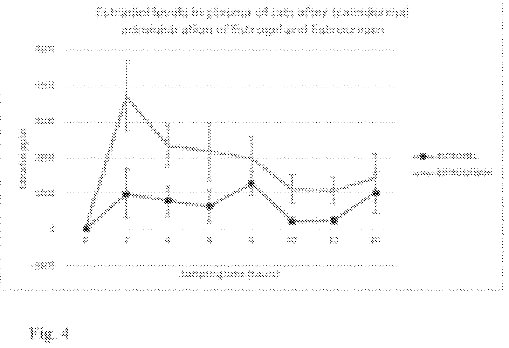

As is evident from the results shown in Fig. 4, the ESTROCREAM mixture surprisingly and unexpectedly proved to be 2.5 fold more bioavailable than the ESTROGEL^{®} product in this rat model despite a lower rate of permeation.

These results clearly show significant differences between the two products Tmax, Cmax, and bioavailability. Kinetic data is summarized in table 3.

**Table 3**

| | ESTROGEL product | ESTROCREAM composition (Example 2) |
|---|---|---|
| Cₘₐₓ | 1,304 (2,455)* | 3,695 (4,416)* |
| Tₘₐₓ | 8H | 2H |

Unlike the hydroalcoholic formulation, a gel composition described herein does not evaporate and drug is not left on the surface of the skin. It can be more efficiently delivered to the stratum corneum reservoir from which there can be controlled delivery of the drug to the vascular dermis and absorption into the systemic circulation.

### Example 3: Comparing bioavailability of an exemplary gel composition with a standard oil and water emulsion.

In order to demonstrate that a gel composition described herein can maximizes drug concentrations in the stratum corneum and the serum and thereby increase bioavailability in a manner that may not be inherent to oil and water emulsions, the following *in-vivo* kinetic study in rats were performed.

As a comparative to the TESTOCREAM mixture (Example I) a 1% testosterone formulation in a standard emulsion base was prepared. The formula is both textbook and virtually identical to marketed products with other active ingredients (To the inventor's knowledge no commercial transdermal testosterone emulsion product exists).

### Test Emulsion formula (%w/w):

Testosterone 1.0%
Carbomer (Carbopol 980 NF) 1.4%
Carbomer (Carbopol 974) 1.0%
Glycerin 12.0%
Mineral Oil 6.0%
Alcohol 96.5° 3.5%
Sodium Methyl Paraben 0.25%
Sorbic Acid 0.1%
Purified Water USP/EP 74.75%
Total 100%

The test formulation was manufactured in the standard way, by dissolving the testosterone in the alcohol and then incorporated with the mineral oil in the oil phase. The carbomers will be hydrated in the aqueous phase. After hydration glycerin, Sorbic acid and Sodium Methyl paraben will be added to the aqueous phase. The two phases will then be mixed in an appropriate vessel.

Using the same method as described in example 1, the different compositions were applied to two groups of rats which received transdermally the same quantity of testosterone in either the Test Emulsion (above) or TESTOCREAM mixture (Example I). The blood was collected up to 24 h (8 time points) and testosterone levels were determined.

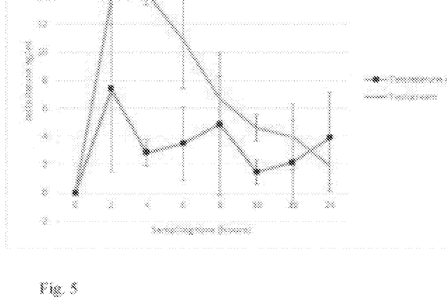

As is evident from the results shown in Fig. 5, the TESTOCREAM composition proved to be significantly more bioavailable than the test Emulsion in this rat model, and it can accordingly be concluded that even though it may be possible to provide a reservoir in the stratum corneum with a gel composition described herein, it may not be possible with a standard oil and water emulsion.

Thus, a gel composition described herein can be arranged for maximizing drug concentrations in the stratum corneum and the serum, and thereby increases bioavailability.

### Example 4: Preparing an exemplary gel composition comprising diclofenac diethylamine.

An exemplary gel composition, called DICLOCREAM herein, with the following composition was prepared in a manner similar to the TESTOCREAM and ESTROCREAM compositions.

### DICLOCREAM composition (%w/w):

Diclofenac 1%
Carbomer (CARBOPOL^{®} 980 NF) 1.4%
Sorbic Acid 0.2%
Ethanol 96.5° 3.5%
Sodium methyl Paraben 0.25
EMULFREE^{®} P 4%
Purified Water (USP/EP) 69.565%
LABRAFAC^{®} lipophile WL 1349 20%
Sodium Hydroxide 0.085%

Diclofenac diethylamine is a Non-Steroidal Anti-Inflammatory drug (NSAID), that can be typically used in the treatment of inflammation and degenerative disorders of the musculoskeletal system.

The conventional diclofenac emulgel formulation contains isopropyl alcohol to increase solubility of diclofenac diethylamine, it is highly flammable and may cause eye and cutaneous irritation. Prolonged skin contact with isopropyl alcohol may cause eczema and sensitivity.

The DICLOCREAM composition differs from the currently available commercial product in several ways. The DICLOCREAM composition can be safer as it only has a 3.5 % alcohol content vs. flammable levels of isopropyl alcohol in the conventional products. This means that the DICLOCREAM composition can be taken on an aircraft whereas traditional flammable formulations should not be carried on board an aircraft. The DICLOCREAM composition can be more user friendly, in that much less product may be required (~40 % less than lead competitors), and may be quicker drying (no need to stand and dry). Thus, the DICLOCREAM product has better patient acceptability since it may be neither watery, messy nor smelly. Surprisingly and unexpectedly, the DICLOCREAM product has improved bioavailability despite a lower permeation rate as seen with TESTOCREAM and ESTROCREAM, and thus maximizes the concentration of diclofenac in the affected joint.

The gel composition can be inexpensive to manufacture, and due to the ease of application it can be used equally well both privately and in medical or hospital facilities.

### Example 5: Preparing other exemplary gel compositions

Composition similar to TESTOCREAM and ESTROCREAM can be prepared with micronized active ingredients in order to improve the solubility of the active ingredient

### Exemplary composition comprising micronized testosterone (%w/w):

Micronized Testosterone 1.0%
Carbomer (CARBOPOL ^{®} 980 NF) 1.4%
Sorbic Acid 0.2%
Ethanol 96.5° 3.5%
Sodium Methyl Paraben 0.25%
EMULFREE^{®} P 4.0%
Purified water USP/EP 69.57%
LABRAFAC^{®} lipophilic WL 1349 20.0%
Sodium Hydroxide 0.08%
Total 100%

### Exemplary composition comprising micronized estradiol (%w/w):

Micronized Estradiol 0.1%
Ethanol 96.5° 5.0%
LABRAFAC0 lipophile WL1349 19.40%
EMULFREE0 P 4.0%
Purified water (1) 62.95%
CARBOPOL0 980 F 1.4%
Purified water (2) 3.3%
Sodium methylparaben 0.25%
Purified water (3) 3.315%
Sodium hydroxide 0.085%
Sorbic acid 0.2%

### Exemplary composition comprising micronized diclofenac (%w/w):

Micronized Diclofenac 1%
Carbomer (CARBOPOL^{®} 980 NF) 1.4%
Sorbic Acid 0.2%
Ethanol 96.5° 3.5%
Sodium methyl Paraben 0.25
EMULFREE^{®} P 4%
Purified Water (USP/EP) 69.565%
LABRAFAC^{®} lipophile WL 1349 20%
Sodium Hydroxide 0.085%

Gel compositions containing other active ingredients can be prepared in a similar fashion to the formulations described above. These active ingredients can include other hormones such as ethinylestradiol; levonorgestrel, salts of any of these; and micronized forms of any of these.

### Example 6: Moisturizing formulations comprising a bioadhesive

An exemplary moisturizing gel composition containing a bioadhesive was prepared in a manner similar to the TESTOCREAM and ESTROCREAM compositions.

### Bioadhesive formulation (%w/w):

Carbomer (Carbopol 974P NF) 1.0%
Polycarbophil Noveon AA1 (USP) 1.5%
Sorbic Acid (USP/EP) 0.1%
Labrafac Lipophile WL 1349 (USP/EP) 17.07%
EmulFree P Pharma Grade 2.0%
Purified Water (USP/EP) 76.4%
Sodium Hydroxide (USP/EP) QS AD pH 2.8 -3.2
Purified Water (USP/EP) QS 100%

A study was conducted at the Universite d'Auverngne on the effect of Replens^{®} vs. the formulation of Example 5 on epithelial cells *in vitro.*

Mucoadhesion (Bioadhesion) studies were conducted on piglet (15-20 Kg) jejunal mucosa. After anesthesia, intestinal tissue from jejunum (40 cm length) was taken, placed in cold normal saline (0.9 % NaCl) in order to avoid cellular alterations. Then tissue was cut into equal fragments of 4 cm and open longitudinally (about 10 cm²). Each fragment was mounted on a Petri dish and 2 g of formulations (Replens MD or Example 6) were applied on mucosal side.

Then 8 mL of phosphate buffer saline (PBS) were put in Petri dish and incubated in incubator at 37 °C, with a relative humidity > 80 % and C0₂ 5 %. After lh incubation, tissue was taken off and placed in 50 mL centrifugal tube with 4 mL of normal saline (0.9 % NaCl) solution. Each tube was shaken with a vortex for 15s. 100 µL were sampled and 100 µL of trypan blue (0.4 % m/v in PBS) were added. After 5 min incubation, 10 µL were transferred to the cut-out notch on the slide chamber of Kova Glasstic Slide 10.

By capillary action 6.6 µL of the sample were drawn into the Kova Slide chamber resulting in a homogenous suspension of the sediment. Finally, clumping dead cells (desquamating cells) were counted. In this test, the agglutination of at least 5 desquamating cells is considered as cells cluster.

**Table 4: Number of Cells clumping/ µL Replens vs. Example 5 and Control**

| | | | | | |
|---|---|---|---|---|---|
| Replens 1 | 260 | Example 6-1 | 160 | Control 1 | 60 |
| Replens 2 | 280 | Example 6-2 | 140 | Control 2 | 40 |
| Replens 3 | 180 | Example 6-3 | 100 | Control 3 | 0 |
| Replens 4 | 240 | Example 6-4 | 80 | Control 4 | 80 |
| Replens 5 | 300 | Example 6-5 | 80 | Control 5 | 60 |
| Replens 6 | 180 | Example 6-6 | 40 | Control 6 | 60 |
| Mean | 240 | | 100 | | 50 |
| SD | 51 | | 44 | | 28 |
| CV | 21 | | 44 | | 55 |

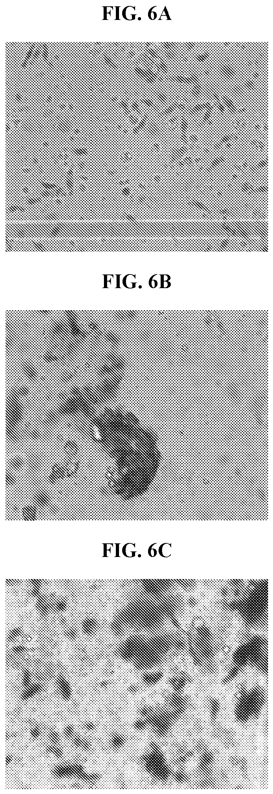

Figure 6A depicts an exemplary microscopic examination of a piglet administered a control formulation. In the control group a few cells were dead but no cell clumping was observed. Figure 6B depicts an exemplary microscopic examination of a piglet administered Replens. In the Replens group many clusters of dead cells have been observed. Figure 6C depicts an exemplary microscopic examination of a piglet administered the gel formulation of Example 6. Surprisingly and unexpectedly, only a few cell clumps were observed. The dead cells are well individualized. Moreover, no cell is recovered in the large blue clusters in contrast to the Replens formulation.

### Example 7: Vaginal delivery formulations comprising a bioadhesive

The following exemplary gel compositions containing a bioadhesive can be prepared in a manner similar to the TESTOCREAM, ESTROCREAM, Example 5, and Example 6 compositions.

### Progesterone formulation (%w/w): (not part of the invention)

| | |
|---|---|
| Micronized Progesterone (USP/EP) | 8.0% |
| Carbomer (Carbopol 974P NF) | 1.0% |
| Polycarbophil Noveon AA1 (USP) | 1.5% |
| Sorbic Acid (USP/EP) | 0.1% |
| Labrafac Lipophile WL 1349 (USP/EP) | 17.07% |
| EmulFree P Pharma Grade | 2.0% |
| Purified Water (USP/EP) | 68.4% |
| Sodium Hydroxide (USP/EP) QS AD pH 2.8 -3.2 Purified Water (USP/EP) | QS 100% |

In addition to progesterone P(4) any synthetic progestin could be formulated utilizing described formulation.

### Estradiol formulation: (%w/w)

| | |
|---|---|
| Micronized Estradiol (USP/EP) | 0.1% |
| Carbomer (Carbopol 974P NF) | 1.0% |
| Polycarbophil Noveon AA1 (USP) | 1.5% |
| Sorbic Acid (USP/EP) | 0.1% |
| Labrafac Lipophile WL 1349 (USP/EP) | 17.07% |
| EmulFree P Pharma Grade | 2.0% |
| Purified Water (USP/EP) | 76.4% |
| Sodium Hydroxide (USP/EP) QS AD pH 2.8 -3.2 Purified Water (USP/EP) | QS 100% |

Any estrogen could be substituted for estradiol for example, dienestrol, estriol, estrone etc.

### Danazol formulation: (%w/w) (not part of the invention)

| | |
|---|---|
| Danazol | 6.67% |
| Carbomer (Carbopol 974P NF) | 1.0% |
| Polycarbophil Noveon AA1 (USP) | 1.5% |
| Methylparaben | 0.25% |
| Labrafac Lipophile WL 1349 (USP/EP) | 15% |
| EmulFree P Pharma Grade | 2.0% |
| Purified Water (USP/EP) | 73.58% |

As discussed in Example 5, gel compositions containing a bioadhesive and other active ingredients can be prepared in a similar fashion to the formulations described above. These active ingredients can include other hormones such as ethinylestradiol; levonorgestrel, salts of any of these; and micronized forms of any of these.

While embodiments have been shown and described herein, it will be obvious to those skilled in the art that such embodiments are provided by way of example only.

## Claims

1. A gel composition comprising:
(a) at least one active ingredient or a salt thereof;
(b) at least one oleogel comprising at least one oily agent and at least one lipid soluble cellulose polymer, wherein the at least one oily agent comprises from about 5% to about 40% by weight of the total weight of the composition and the at least one cellulose polymer comprises from about 1%) to about 10%) by weight of the total weight of the composition; and
(c) at least one aqueous gel; and
wherein: the active agent is micronized; and wherein the at least one active ingredient is dispersed or suspended in at least a portion of the at least one aqueous gel, and wherein the active ingredient is selected from estradiol or a salt thereof, testosterone or a salt thereof, levonorgestrel or a salt thereof, ethinylestradiol or a salt thereof, and/or diclofenac or a salt thereof.

2. The gel composition of claim 1 wherein the composition comprises a bioadhesive.

3. The gel composition of claim 1 or 2 wherein the composition comprises at most about 4% w/w of an alcohol, wherein the alcohol is a C₁-C₈ alcohol.

4. The gel composition of claim 1, wherein the lipid soluble cellulose polymer is an alkyl cellulose, e.g. selected from the group consisting of methylcellulose, ethylcellulose, hydroxypropylcellulose, and combinations thereof, or wherein the lipid soluble cellulose polymer is an alkyl carboxylic containing cellulose or a salt thereof, e.g. a non-sodium containing carboxymethylcellulose.

5. The gel composition of claim 1, wherein the oily agent is selected from the group consisting of: a synthetic diglyceride; a synthetic triglyceride; a propylene glycol isostearate; a polyoxy ethyl enated oleic glyceride mixture; an oil of plant origin; and any combination thereof.

6. The gel composition of claim 1, wherein the composition comprises no more than about 2% of a surfactant by weight of the total weight of the composition, wherein the surfactant is selected from the group consisting of non-ionic, cationic, amphoteric, zwitterionic, and any combination thereof.

7. The gel composition of claim 1, wherein the aqueous gel further comprises at least one gelling agent, e.g. a carbomer; a poloxamer; sodium carboxymethylcellulose; and a combination thereof, and wherein the at least one gelling agent preferably comprises from about 0.1% to about 10% by weight of the total weight of the aqueous gel.

8. The gel composition of claim 1 comprising the bioadhesive, wherein the bioadhesive is selected from the group consisting of: a carbomer; glyceryl monooleate; hypromellose; polycarbophil; poly(methylvinyl ether-co-maleic anhydride); a salt thereof; and a combination thereof.

9. The gel composition of any one of claims 1-9, wherein the composition is in unit dose form.

10. A method of preparing a gel composition comprising:
(a) dispersing at least one micronized active ingredient in an aqueous gel;
(b) contacting the dispersion of (a) with at least one oleogel gel to form an a gel composition;
wherein the at least one oleogel comprises at least one oily agent and at least one lipid soluble cellulose polymer, and wherein the active ingredient is selected from estradiol or a salt thereof, testosterone or a salt thereof, levonorgestrel or a salt thereof, ethinylestradiol or a salt thereof, and/or diclofenac or a salt thereof.

11. The method of claim 10, wherein the at least one oily agent comprises from about 5% to about 40% by weight of the total weight of the composition and the at least one cellulose polymer comprises from about 1% to about 10% by weight of the total weight of the composition.

12. The method of claim 11, further comprising dissolving an alcohol in at least a portion of the at least one aqueous gel, at least one oleogel, or a combination thereof; wherein the amount of alcohol is at most about 4% w/w, and wherein the alcohol preferably is ethanol or isopropanol.

13. The method of claim 10, further comprising dispersing a bioadhesive into at least a portion of the aqueous gel, oleogel, or a combination thereof.

14. Gel composition according to claims 1 - 9 for use in at least partially ameliorating a condition by applying the gel composition to at least a portion of a skin of a subject, and wherein the condition is selected from the group consisting of a hormone imbalance, menopause, endometriosis, cancer, dry skin, arthritis, and a migraine.

## Patentansprüche

1. Gelzusammensetzung, umfassend:
(a) mindestens einen Wirkstoff oder ein Salz davon;
(b) mindestens ein Oleogel, umfassend mindestens ein öliges Mittel und mindestens ein lipidlösliches Cellulosepolymer, wobei das mindestens eine ölige Mittel von etwa 5 Gew.-% bis etwa 40 Gew.-% des Gesamtgewichts der Zusammensetzung ausmacht und das mindestens eine Cellulosepolymer von etwa 1 Gew.-% bis etwa 10 Gew.-% des Gesamtgewichts der Zusammensetzung ausmacht; und
(c) mindestens ein wässriges Gel; und
wobei: der Wirkstoff mikronisiert ist; und wobei der mindestens eine Wirkstoff in mindestens einem Teil des mindestens einen wässrigen Gels dispergiert oder suspendiert ist, und wobei der Wirkstoff ausgewählt ist aus Estradiol oder einem Salz davon, Testosteron oder einem Salz davon, Levonorgestrel oder einem Salz davon, Ethinylestradiol oder einem Salz davon und/oder Diclofenac oder einem Salz davon.

2. Gelzusammensetzung nach Patentanspruch 1, wobei die Zusammensetzung einen Bioklebstoff umfasst.

3. Gelzusammensetzung nach Patentanspruch 1 oder 2, wobei die Zusammensetzung höchstens etwa 4 Gew.-% eines Alkohols umfasst, wobei der Alkohol ein C₁-C₈-Alkohol ist.

4. Gelzusammensetzung nach Patentanspruch 1, wobei das lipidlösliche Cellulosepolymer eine Alkylcellulose ist, z.B. ausgewählt aus der Gruppe bestehend aus Methylcellulose, Ethylcellulose, Hydroxypropylcellulose und Kombinationen davon, oder wobei das lipidlösliche Cellulosepolymer eine alkylcarboxylhaltige Cellulose oder ein Salz davon ist, z.B. eine nicht natriumhaltige Carboxymethylcellulose.

5. Gelzusammensetzung nach Patentanspruch 1, wobei das ölige Mittel ausgewählt ist aus der Gruppe bestehend aus: einem synthetischen Diglycerid; einem synthetischen Triglycerid; einem Propylenglycolisostearat; einer polyoxyethylenierten Ölglyceridmischung; einem Öl pflanzlichen Ursprungs; und einer beliebigen Kombination davon.

6. Gelzusammensetzung nach Patentanspruch 1, wobei die Zusammensetzung nicht mehr als etwa 2 Gew.-% eines Tensids des Gesamtgewichts der Zusammensetzung umfasst, wobei das Tensid ausgewählt ist aus der Gruppe bestehend aus nichtionisch, kationisch, amphoter, zwitterionisch und einer beliebigen Kombination davon.

7. Gelzusammensetzung nach Patentanspruch 1, wobei das wässrige Gel ferner mindestens ein Geliermittel umfasst, z.B. ein Carbomer; ein Poloxamer; Natriumcarboxymethylcellulose; und eine Kombination davon, und wobei das mindestens eine Geliermittel vorzugsweise von etwa 0,1 Gew.-% bis etwa 10 Gew.-% des Gesamtgewichts des wässrigen Gels ausmacht.

8. Gelzusammensetzung nach Patentanspruch 1, umfassend den Bioklebstoff, wobei der Bioklebstoff ausgewählt ist aus der Gruppe bestehend aus: einem Carbomer; Glycerylmonooleat; Hypromellose; Polycarbophil; Poly(methylvinylether-co-maleinsäureanhydrid); einem Salz davon; und einer Kombination davon.

9. Gelzusammensetzung nach einem der Patentansprüche 1-9, wobei die Zusammensetzung in Einheitsdosisform vorliegt.

10. Verfahren zur Herstellung einer Gelzusammensetzung, umfassend:
(a) Dispergieren mindestens eines mikronisierten Wirkstoffs in einem wässrigen Gel;
(b) Inkontaktbringen der Dispersion von (a) mit mindestens einem Oleogelgel, um eine Gelzusammensetzung zu bilden;
wobei das mindestens eine Oleogel mindestens ein öliges Mittel und mindestens ein lipidlösliches Cellulosepolymer umfasst, und wobei der Wirkstoff ausgewählt ist aus Estradiol oder einem Salz davon, Testosteron oder einem Salz davon, Levonorgestrel oder einem Salz davon, Ethinylestradiol oder einem Salz davon und/oder Diclofenac oder einem Salz davon.

11. Verfahren nach Patentanspruch 10, wobei das mindestens eine ölige Mittel von etwa 5 Gew.-% bis etwa 40 Gew.-% des Gesamtgewichts der Zusammensetzung ausmacht und das mindestens eine Cellulosepolymer von etwa 1 Gew.-% bis etwa 10 Gew.-% des Gesamtgewichts der Zusammensetzung ausmacht.

12. Verfahren nach Patentanspruch 11, ferner umfassend das Auflösen eines Alkohols in mindestens einem Teil des mindestens einen wässrigen Gels, mindestens eines Oleogels oder einer Kombination davon; wobei die Menge an Alkohol höchstens etwa 4 Gew.-% beträgt, und wobei der Alkohol vorzugsweise Ethanol oder Isopropanol ist.

13. Verfahren nach Patentanspruch 10, ferner umfassend das Dispergieren eines Bioklebstoffs in mindestens einem Teil des wässrigen Gels, Oleogels oder einer Kombination davon.

14. Gelzusammensetzung nach den Patentansprüchen 1 - 9 zur Verwendung bei der mindestens teilweisen Verbesserung eines Zustands durch Auftragen der Gelzusammensetzung auf mindestens einen Teil einer Haut eines Subjekts, und wobei der Zustand ausgewählt ist aus der Gruppe bestehend aus einem Hormonungleichgewicht, Menopause, Endometriose, Krebs, trockener Haut, Arthritis und einer Migräne.

## Revendications

1. Une composition sous forme de gel, qui comprend :
(a) au moins un ingrédient actif ou un sel de ce dernier ;
(b) au moins un oléogel qui comprend au moins un agent huileux et au moins un polymère cellulosique soluble dans les lipides ; dans lequel ledit au moins un agent huileux comprend d'environ 5 % à environ 40 % en poids du poids total de la composition et ledit au moins un polymère cellulosique comprend d'environ 1 % à environ 10 % en poids du poids total de la composition ; et
(c) au moins un gel aqueux ; et
dans laquelle : l'agent actif est micronisé ; et dans lequel ledit au moins un ingrédient actif est mis en dispersion ou est mis en suspension dans au moins une portion dudit au moins un gel aqueux ; et dans laquelle l'ingrédient actif est choisi parmi l'œstradiol ou un de ses sels, la testostérone ou un de ses sels, le lévonorgestrel ou un de ses sels, l'éthinyloestradiol ou un de ses sels, et/ou le diclofénac ou un de ses sels.

2. La composition sous forme de gel selon la revendication 1, dans laquelle la composition comprend un bioadhésif.

3. La composition sous forme de gel selon la revendication 1 ou 2, dans laquelle la composition comprend au maximum environ 4 % en poids/poids d'un alcool ; dans lequel l'alcool est un alcool en C₁-C₈.

4. La composition sous forme de gel selon la revendication 1, dans laquelle le polymère cellulosique soluble dans les lipides est une alkylcellulose, par exemple choisie parmi le groupe constitué par la méthylcellulose, l'éthylcellulose, l'hydroxypropylcellulose et leurs combinaisons, ou dans laquelle le polymère cellulosique soluble dans les lipides est une cellulose contenant un composé alkylcarboxylique ou un de ses sels, par exemple une carboxyméthylcellulose qui ne contient pas du sodium.

5. La composition sous forme de gel selon la revendication 1, dans laquelle l'agent huileux est choisi parmi le groupe constitué par : un diglycéride synthétique ; un triglycéride synthétique ; un isostéarate de propylène glycol ; un mélange de glycérides oléiques contenant du polyoxyéthylène ; une huile d'origine végétale ; ainsi que l'une quelconque de leurs combinaisons.

6. La composition sous forme de gel selon la revendication 1, dans laquelle la composition ne comprend pas plus d'environ 2 % d'un agent tensioactif en poids du poids total de la composition ; dans laquelle l'agent tensioactif est choisi parmi le groupe constitué par un agent tensioactif non ionique, cationique, amphotère, zwitterionique, ainsi que l'une quelconque de leurs combinaisons.

7. La composition sous forme de gel selon la revendication 1, dans laquelle le gel aqueux comprend en outre au moins un agent gélifiant, par exemple un carbomère ; un poloxamère ; de la carboxyméthylcellulose de sodium ; et une de leurs combinaisons ; et dans laquelle ledit au moins un agent gélifiant comprend de préférence d'environ 0,1 % à environ 10 % en poids du poids total du gel aqueux.

8. La composition sous forme de gel selon la revendication 1, qui comprend le bioadhésif ; dans laquelle le bioadhésif est choisi parmi le groupe constitué par : un carbomère ; du monooléate de glycéryle ; de l'hypromellose ; du polycarbophile ; un copolymère d'éther polyméthylvinylique et d'anhydride maléique ; un de leurs sels ; et une de leurs combinaisons.

9. La composition sous forme de gel selon l'une quelconque des revendications 1 à 9, dans laquelle la composition se présente sous une forme posologique unitaire.

10. Un procédé de préparation d'une composition sous forme de gel, qui comprend :
(a) la mise en dispersion d'au moins un ingrédient actif micronisé dans un gel aqueux ;
(b) la mise en contact de la dispersion de (a) avec au moins un gel sous forme d'oléogel afin d'obtenir une composition sous forme de gel ;
dans lequel ledit au moins un oléogel comprend au moins un agent huileux et au moins un polymère cellulosique soluble dans les lipides, et dans lequel l'ingrédient actif est choisi parmi l'œstradiol ou un de ses sels, la testostérone ou un de ses sels, le lévonorgestrel ou un de ses sels, l'éthinyloestradiol ou un de ses sels, et/ou le diclofénac ou un de ses sels.

11. Le procédé selon la revendication 10, dans lequel ledit au moins un agent huileux comprend d'environ 5 % à environ 40 % en poids du poids total de la composition et ledit au moins un polymère cellulosique comprend d'environ 1 % à environ 10 % en poids du poids total de la composition.

12. Le procédé selon la revendication 11, qui comprend en outre la dissolution d'un alcool dans au moins une portion dudit au moins un gel aqueux, dudit au moins un oléogel, ou d'une de leurs combinaisons ; dans lequel la quantité de l'alcool s'élève au maximum à environ 4 % en poids/poids ; et dans lequel l'alcool est de préférence l'éthanol ou l'isopropanol.

13. Le procédé selon la revendication 10, qui comprend en outre la mise en dispersion d'un bioadhésif dans au moins une portion du gel aqueux, de l'oléogel, ou d'une de leurs combinaisons.

14. Composition sous forme de gel selon les revendications 1 à 9, pour son utilisation dans l'amélioration au moins partielle d'une affection par l'intermédiaire de l'application de la composition sous forme de gel sur au moins une portion d'une peau d'un sujet ; et dans lequel l'affection est choisie parmi le groupe constitué par un déséquilibre hormonal, la ménopause, l'endométriose, un cancer, une peau sèche, l'arthrite et une migraine.
